(19) Europäisches Patentamt
European Patent Office
Office européen des brevets

(11) Veröffentlichungsnummer: **0 061 629**
**B1**

(12) # EUROPÄISCHE PATENTSCHRIFT

(45) Veröffentlichungstag der Patentschrift: **29.07.87**

(21) Anmeldenummer: **82102028.6**

(22) Anmeldetag: **13.03.82**

(51) Int. Cl.[4]: **C 07 C 53/19,** C 07 C 53/21, C 07 C 53/50, C 07 C 51/00, C 07 C 51/58, C 07 C 69/63, C 07 C 67/00, C 07 C 21/04, C 07 C 17/26 // C07C62/16, C07C51/04, C07C21/18, C07C17/20, C07C69/653, C07C69/743, C07C67/347, C07C67/317

(54) **Verfahren zur Herstellung von substituierter alpha-Halogenpropionsäure und ihren Derivaten; substituiertes Vinylidenchlorid.**

(30) Priorität: **26.03.81 DE 3111848**

(43) Veröffentlichungstag der Anmeldung:
**06.10.82 Patentblatt 82/40**

(45) Bekanntmachung des Hinweises auf die Patenterteilung:
**29.07.87 Patentblatt 87/31**

(84) Benannte Vertragsstaaten:
**BE CH DE FR GB IT LI NL**

(56) Entgegenhaltungen:
**FR-A-1 330 693**
**US-A-2 515 306**
**US-A-2 557 779**
**US-A-3 651 019**
**US-A-3 742 047**
**CHEMICAL ABSTRACTS, Band 54, Nr. 2, 25. Januar 1960, Spalte 1333b, Columbus, Ohio, US, R.Kh. FREIDLINA et al.: "Synthesis of D,D,-alpha-aminocarboxylic acids from compounds containing the CCl2:CH group"**
**CHEMICAL ABSTRACTS, Band 94, Nr. 21, 25. Mai 1981, Seite 662, Zusammenfassung 174377j, Columbus, Ohio, US**

(73) Patentinhaber: **BAYER AG**
**Konzernverwaltung RP Patentabteilung**
**D-5090 Leverkusen 1 Bayerwerk (DE)**

(72) Erfinder: **Arlt, Dieter, Prof. Dr.**
**Rybnikerstrasse 2**
**D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 061 629 B1

**Beschreibung**

Die vorliegende Erfindung betrifft ein neues Verfahren zur Herstellung von substituierten α-Halogenpropionsäurechloriden sowie neue Weiterverarbeitungsprodukte zu ihrer Herstellung.

Es ist bereits bekannt geworden, daß Vinylidenchloride, die durch Aromaten substituiert sind, sich durch Halogenierung in Ameisensäure in die entsprechend substituierten α-Halogenessigsäurederivate überführen lassen. Diese Reaktion war jedoch bei aliphatisch substituierten Vinylidenchloriden nicht bekannt (vgl. Chemical Abstracts *54*; 1333 (1960)).

Es wurde ein neues Verfahren zur Herstellung von substituierten α-Halogenpropionsäurechloriden der Formel I

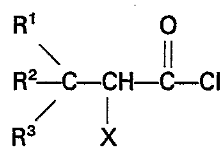

I

gefunden,

in welcher

$R^1$ für Halogen Alkyl; halogensubstituiertes Alkyl, Aryl steht und

$R^2$ und $R^3$ unabhängig voneinander für H, Halogen, Methyl, Ethyl stehen und

X für Cl oder Er steht.

Des neue Verfahren besteht darin, daß man substituierte Vinylidenchloride der Formel II

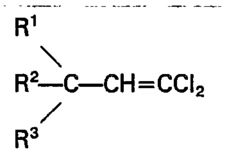

II

in welcher

$R^1$—$R^3$ die oben angegebene Bedeutung besitzen,

mit Chlor oder Chlorbrom in Gegenwart von Verbindungen oder Formel III

$$R^5—SO_3H \qquad III$$

in welcher

$R^5$ für gegebenenfalls halogensubstituiertes $C_{1-18}$-Alkyl oder gegebenenfalls alkylsubstituiertes Aryl steht,

umsetzt.

2. Es würde ferner des neue substituierte α-Halogenpropionsäurechlorid der Formel Ia

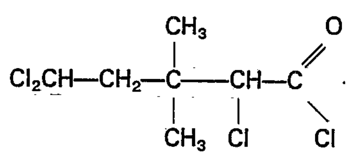

Ia

gefunden

3. Es wurde ferner des neue substituierte Vinylidenchlorid der Formel IV gefunden,

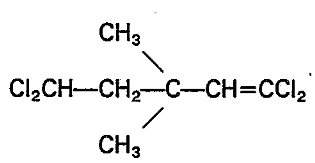

IV

4. Es wurde ferner ein Verfahren zur Herstellung des neuen substituierten Vinylidenchlorids der Formel IV

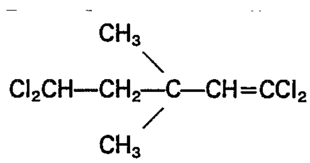

IV

gefunden, das dadurch gekennzeichnet ist, daß man 1,3,3,3-Tetrachlor-1,1-dimethyl-propan mit Vinylchlorid in Gegenwart von Lewis-Säuren umsetzt.

5. Ferner wurde die substituierte α-Halogenpropionsäure der Formel 1b

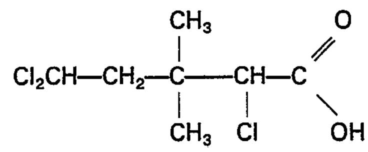

Ib

gefunden.

Es war überraschend, daß das erfindungsgemäße Verfahren gemäß 1 (oben) universell anwendbar ist und mit guten Ausbeuten verläuft wenn man, nicht wie aus dem Stand der Technik bekannt in Ameisensäure, sondern in Gegenwart der Sulfonsäure und ihrer Ester der Formel III (oben) arbeitet. Diese Arbeitsweise hat außerdem den Vorzug, daß die als Reaktionsmedium dienenden Sulfonsäuren und ihre Ester leicht wieder zurückgewonnen werden können.

Das erfindungsgemäße Verfahren gemäß 1 (oben) kann, wenn von 1,1,5,5-Tetrachlor-3,3-dimethyl-pent-1-en ausgegangen wird, durch folgendes Formelschema dargestellt werden:

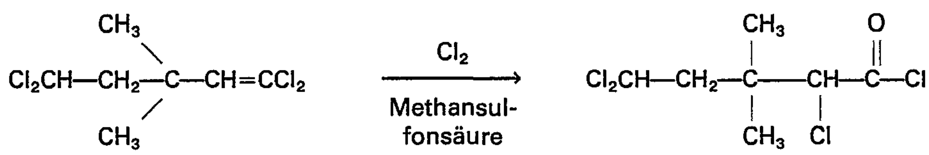


Nach dem erfindungsgemäßen Verfahren werden bevorzugt Verbindungen der Formel I herstellt in welcher

X für Cl steht;

$R^1$ für H, $C_{1-13}$-Alkyl, das gegebenenfalls durch F, Cl, Br substituiert ist, steht und insbesondere für H, $C_{1-13}$-Alkyl, $C_{1-4}$-Alkyl das durch 1—2 Cl- oder F-Reste substituiert ist, steht;

$R^2$ für H, Cl oder Methyl steht.

Besonders bevorzugt sind neben den in den Beispielen genannten Verbindungen der Formel I die folgenden Verbindungen:

2,5-Dichlor-3,3-dimethyl-pentansäurechlorid, 2-Chlor-5-fluor-3,3-dimethyl-pentensäurechlorid, 2,5,5-Trichlor-3,3-dimethyl-pentansäurechlorid, 2-Brom-5,5-dichlor-3,3-dimethyl-pentansäurechlorid, 2-Chlor-n-2-brom-3-methylbutansäurechlorid, 2-Brom-3,3-dimethyl-butansäurechlorid.

Das erfindungsgemäße Verfahren gemäß 1 (oben) wird zwischen −20°C bis 100°C, bevorzugt zwischen 0 und 50°C durchgeführt.

Es wird bei Normaldruck oder leichtem Überdruck gearbeitet.

Das Halogenierungsmittel wird in bezug auf die eingesetzten Vinylidenchloride der Formel II in äquivalenter Menge, d.h. auf 1 Mol der Verbindung der Formel II 1 Mol Chlor oder 2 Mol Chlorbrom.

Verbindungen der Formel III in deren Gegenwart das erfindungsgemäße Verfahren durchgeführt wird, sind Sulfonsäuren wie Methansulfonsäure, Ethansulfonsäure, Chlormethansulfonsäure, Benzolsulfonsäure, o- und -Toluolsulfonsäure, i-Dodecyl-benzolsulfonsäure.

Besonders bevorzugt ist das Arbeiten in Gegenwart von Methansulfonsäure.

Das erfindungsgemäße Verfahren wird bevorzugt ohne Lösungsmittel durchgeführt. Man kann jedoch auch in Gegenwart von Lösungsmitteln arbeiten, die gegenüber den verwendeten Halogenierungsmitteln inert sind. Als solche seien z.B. genannt Chlorkohlenwasserstoffe wie Dichlormethan oder Dichlorethan.

Das erfindungsgemäße Verfahren wird so durchgeführt, daß man die Verbindungen der Formel II, gegebenenfalls gelöst in einem gegen Halogene inerten Lösungsmittel, mit den Verbindungen der Formel III mischt und in dieses Gemisch Chlor einleitet. Das Ende der Reaktion wird dadurch bestimmt, daß eine Probe des Reaktionsgemisches mit Hexan extrahiert und der Extrakt IR-spektrokopisch untersucht wird. Das Verschwinden der Absorptionsbanden bei 1600 $cm^{-1}$, die für Verbindungen der Formel I charakterisiert ist, zeigt das Ende der Reaktion an.

Zur Abführung überschüssiger Reaktionswärme ist es u.U. erforderlich zu kühlen.

Die Isolierung der Verbindungen der Formel I kann durch Extraktion z.B. mit Kohlenwasserstoffen wie Pentan, Hexan oder Leichtbenzin erfolgen. Die Verbindungen der Formel I können aber auch destillativ aus dem Reaktionsgemisch abgetrennt werden.

Soll ein Ester oder eine Säure aus dem Säurechlorid der Formel I hergestellt werden, ist es zweckmäßig, dem Reaktionsgemisch eine mindestens äquivalente Menge eines entsprechenden Alkohol oder Wasser zuzugeben und den Ester oder die Säure durch Extraktion oder Destillation aus dem Reaktionsgemisch zu entfernen.

Das substituierte α-Halogenpropionsäurechlorid der Formel $I_2$ ist neu:

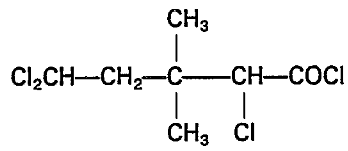

Ia

Ebenfalls neu ist die daraus durch Hydrolyse erhaltene α-Halogenpropionsäure der Formel Ib

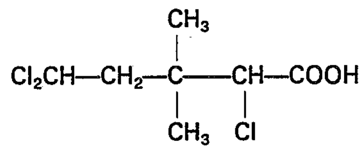

Ib

Die neue Verbindung der Formel I a läßt sich als Herbizid einsetzen. Sie kann aber auch in einem noch nicht zum Stand der Technik gehörenden Verfahren zur bekannten Caronaldehydsäure weiterverarbeitet werden. Dieses Verfahren läßt sich durch folgendes Formelschema wiedergeben:

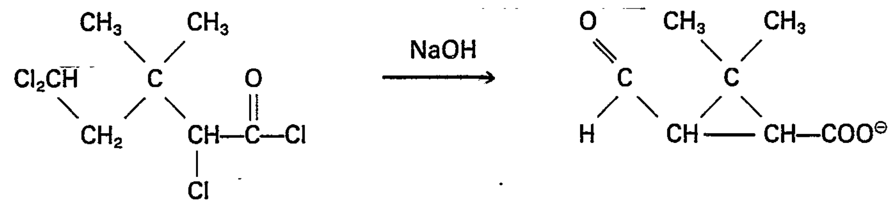


(Ia)

Die Verbindung der Formel Ia wird dazu in wasserhaltigen Lösungsmitteln mit Basen wie Alkali- oder Erdalkalihydroxid behandelt und die entstandenen Salze der Caronaldehydsäure angesäuert und die Säure in üblicher Weise isoliert.

Die neue Verbindung Ia kann auch in einem noch nicht zum Stand der Technik gehörenden Verfahren zur bekannten Permethrinsäure weiterverarbeitet werden. Dieses Verfahren läßt sich durch folgendes Formelschema wiedergeben:

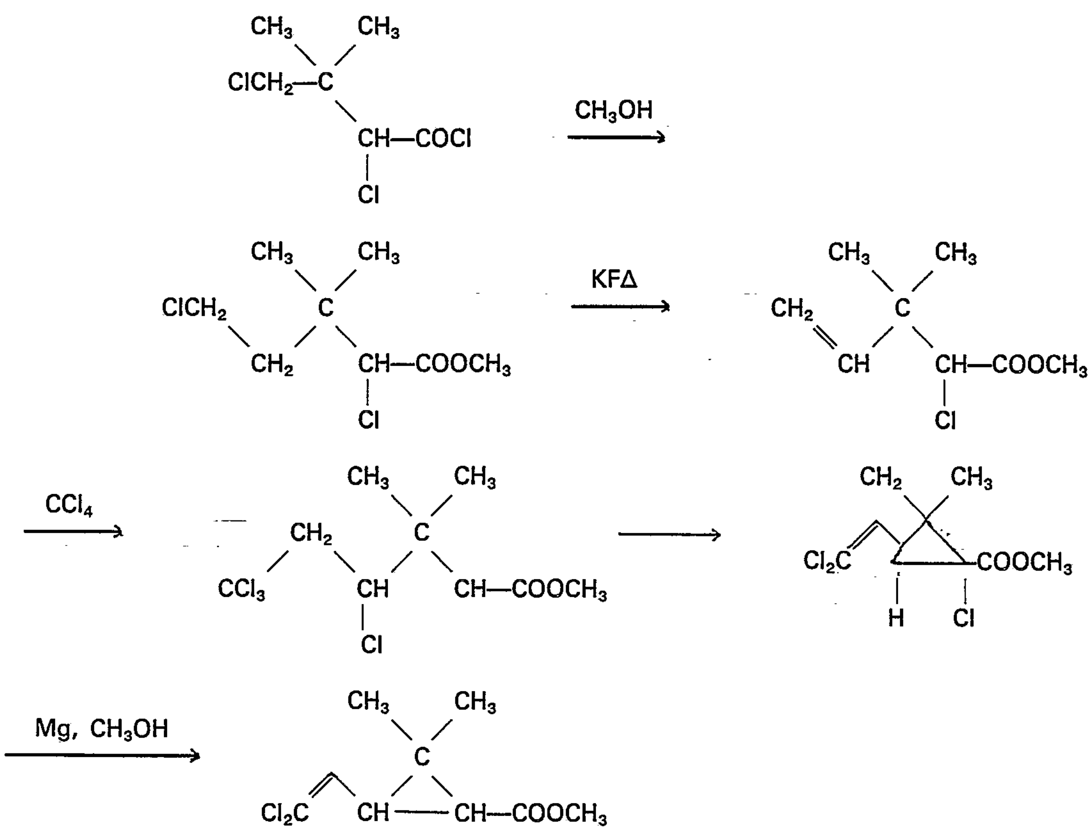


Die einzelnen Verfahrensstufen werden dabei analog zu bekannten Verfahren durchgeführt.

Gegenstand der Erfindung ist ferner das Verfahren zur Herstellung des neuen Vinylidenchlorids gemäß 3 (oben) der Formel IV

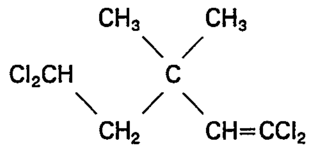

IV

Diese Verbindung wird nach dem erfindungsgemäßen Verfahren gemäß 4 (oben) hergestellt. Dieses Verfahren läßt sich durch folgendes Formelschema wiedergeben:

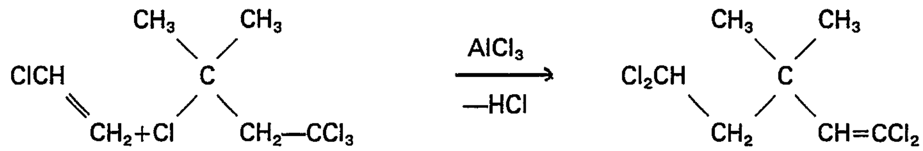


Die Reaktion wird zwischen −20°C und +30°C, bevorzugt zwischen −5 und +30°C durchgeführt. Es wird bevorzugt ohne Lösungsmittel gearbeitet. Doch können auch inerte organische Lösungsmittel wie z.B. Halogenkohlenwasserstoffe. wie Methylenchlorid verwendet werden.

Das Tetrachlordimethylpropen und Vinylchlorid werden in äquimolarem Verhältnis eingesetzt. Es wird in Gegenwart von Lewis-Säuren gearbeitet. Als solche kommen in Frage z.B. $AlCl_3$, $AlBr_3$, $BeCl_2$, $ZnCl_2$, $BF_3$, $TiCl_4$, $SnCl_4$, $SbCl_3$, $FeCl_3$. Bevorzugt werden verwendet: $AlCl_3$, $FeCl_3$, $ZnCl_2$, $TiCl_4$.

Überraschenderweise verläuft die Reaktion in guter Selektivität obwohl Folgereaktionen der entstandenen neuen Vinylidenchloride der Formel II sowie Eliminierungsreaktion sowohl an dem Tetrachlordimethylpropan als auch an dem Vinylidenchlorid zu erwarten gewesen wären.

Die übrigen als Ausgangsmaterialien für das erfindungsgemäße Verfahren gemäß 1 (oben) dienenden Verbindungen der Formel II sind z.T. bekannt bzw. werden analog zu bekannten Verfahren erhalten (vgl. z.B. J. Am. Chem. Soc. 74, 2885 (1952) bzw. eine eigene noch nicht zum Stand der Technik gehörende Anmeldung der Anmelderin DE-Anmeldung P 30 29 270.7, welche EP—A—45 426 entspricht.

Die folgenden Beispiele illustrieren den Gegenstand der vorliegenden Erfindung ohne eine Beschränkung hinsichtlich seiner Breite anzugeben:

Beispiel 1

182 g 1,1,5,5-Tetrachlor-3,3-dimethyl-pent-1-en werden in 400 ml Methansulfonsäure gelöst. Bei 10—20°C (Wasserkühlung) werden 80 g Chlor eingeleitet. Die Reaktion wird solange weitergeführt, bis eine Probe, die durch Extraktion der Reaktionsmischung mit Hexan erhalten wird, keine IR-Absorption bei 1610 $cm^{-1}$ zeigt. Dann wird die Reaktionslösung insgesamt mit Hexan extrahiert. Aus der Hexanphase gewinnt man durch Vakuumdestillation—nach Abtreiben des Hexans—156 g (=80% d. Th.) 2,5,5-Trichlor-3,3-dimethyl-pentansäure-chlorid vom Siedebereich $Kp._{0,12}$92—95°C.

Beispiel 2

Herstellung der Ausgangsprodukte

In eine Lösung von 15 g $AlCl_3$ in 1000 ml Methylenchlorid werden 75 g Vinylchlorid bei −20°C eingeleitet und gleichzeitig 500 g 1,3,3,3-Tetrachlor-1,1-dimethyl-propan der Formel I und weitere 105 g Vinylchlorid zu der Reaktionslösung innerhalb von 180 Minuten zudosiert. Danach läßt man das Reaktionsgemisch 180 Minuten bei −10°C weiterreagieren und versetzt dann die Lösung mit 1000 ml Wasser. Nach der Trennung wird die wäßrige Phase mehrfach mit Methylenchlorid extrahiert, die vereinigten organischen Phasen werden mit Zeolith getrocknet und fraktioniert destilliert. Man erhält 230 g Ausgangsprodukt vom Siedebereich $Kp._{0,1}$ 32—37°C und 270 g 1,1,5,5-Tetrachlor-3,3-dimethyl-pent-1-en der Formel II vom Siederbereich $Kp._{0,15}$ 72—76°C. Dieses Ergebnis entspricht einem Umsetz von 54% mit einer Selektivität von 88%.

Beispiel 3

Weiterverarbeitung des nach Beispiel 1 gewonnenen Produkts zu Caronaldehydsäure

In einem Rührgefäß werden 100 ml Wasser vorgelegt und auf 100°C erhitzt. Dann werden unter pH-Kontrolle gleichzeitig 63 g (0,25 Mol) 2,5,5-Trichlor-3,3-dimethylpentansäurechlorid und eine Lösung von 58 g NaOH in 100 ml Wasser so zugetropft, daß ein pH-Wert im Bereich von 9—10 eingehalten wird. Nach 30 Minuten ist die Reaktion beendet. Die Reaktionslösung wird auf 20°C gekühlt, mit Salzsäure auf pH 2 gestellt und mit Dichlormethan extrahiert. Man erhält nach Abtreiben der Lösungsmittels 36 g rohe Säure und nach Destillation im Vakuum 29,6 g (=83,4%) reine trans-3-Formyl-2,2,-dimethyl-cyclopropan-1-carbonsäure, $Kp._{0,5}$ 125—130°C.

$^1H$—NMR: δ=1,3 (s, 3H) 1,35 (s, 3H) 2,46 (m, 2H) 9,55 (d, aufgespalten, 1H) 10,95 (s, 1H) ppm.

Beispiel 4

Wie in Beispiel 5 beschrieben werden in 1000 g Methansulfonsäure 336 g 1,1,5-Trichlor-3,3-dimethyl-penten mit 210 g Chlor umgesetzt. Das Ende der Umsetzung wird IR-spektroskopisch bestimmt. Danach wird mehrfach mit n-Hexan extrahiert. Durch fraktionierte Destillation der Hexanphase erhält man 252 g 2,5-Dichlor-3,3-dimethyl-pentansäurechlorid, $Kp_{0,2}$ 65—68°C.

Beispiel 5

Wie in den vorstehenden Beispielen beschrieben wird 1,1-Dichlor-3,3-dimethyl-5-fluor-penten (58 g) mit Chlor (39 g) in Methansulfonsäure (193 g) umgesetzt. Nach mehrfacher Extraktion des Reaktionsgemisches mit n-Hexan und fraktionierte Destillation erhält man 49 g 2-Chlor-5-fluor-3,3-dimethyl-pentansäurechlorid, $Kp_{13}$ 75—78°C.

1,1-Dichlor-3,3-dimethyl-5-fluor-penten wird erhalten durch 8-stündiges Erhitzen von 1,1,5-Trichlor-3,3-dimethyl-penten (500 g) mit KF (200 g) in Sulfolan (1500 g) und anschließende fraktionierte Destillation, die Verbindung siedet bei $Kp_{12}$ 60—63°C.

Beispiel 4

Wie in Beispiel 4 angegeben werden in 500 g Methansulfonsäure 153 g 1,1-Dichlor-3,3-dimethyl-buten mit 151 g BrCl umgesetzt. Nach beendeter Reaktion wird mit Hexan extrahiert und destilliv aufgearbeitet. Man erhält 120 g 2-Brom-3,3-dimethyl-butansäurechlorid, Siedebereich $Kp_{15}$ 60—65°C.

**Patentansprüche**

1. Verfahren zur Herstellung von substituierten α-Halogenpropionsäurechloriden der Formel

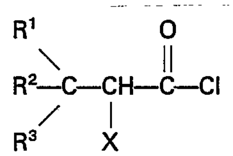

I

in welcher

$R^1$ für H, Halogen, Alkyl; halogensubstituiertes Alkyl, Aryl steht und

$R^2$ und $R^3$ unabhängig voneinander für H, Halogen, Methyl, Ethyl stehen und

X für Cl oder Br steht,

dadurch gekennzeichnet, daß man substituierte Vinylidenchloride der Formel II

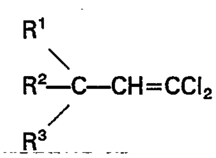

II

in welcher

$R^1$—$R^3$ die oben angegebene Bedeutung besitzen,

bei Normaldruck oder leichtem Überdruck und bei Temperaturen zwischen −20°C und +30°C mit der äquivalenten Menge Chlor oder Chlorbrom in Gegenwart von Verbindungen der Formel III

$$R^5—SO_3H \qquad III$$

in welcher

$R^5$ für gegebenenfalls halogensubstituiertes $C_{1-18}$-Alkyl oder gegebenenfalls alkylsubstituiertes Aryl steht,

umsetzt.

2. Substituiertes α-Halogenpropionsäurechlorid der Formel Ia

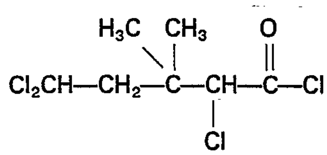

Ia

3. Substituiertes Vinylidenchlorid der Formel IV

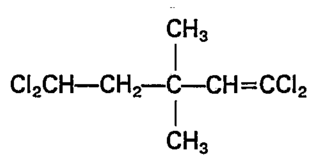

IV

4. Verfahren zur Herstellung des substituierten Vinylidenchlorids der Formel IV

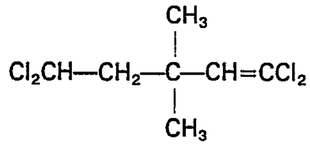

IV

dadurch gekennzeichnet, daß man 1,3,3,3-Tetrachlor-1,1-dimethyl-propan mit der äquivalenten Menge Vinylchlorid in Gegenwart von Lewis-Säuren umsetzt.

5. Substituierte α-Halogenpropionsäure der Formel Ib

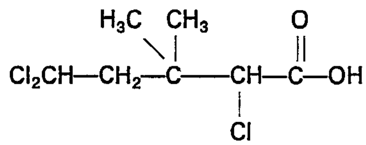

Ib

**Revendications**

1. Procédé de production de chlorures d'acides α-halogénopropioniques substitués de formule

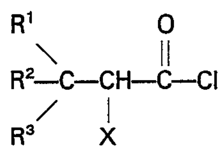

I

dans laquelle
$R^1$ représente H, un halogène, un groupe alkyle; un groupe alkyle halogéné, un groupe aryle et $R^2$ et $R^3$ représentent, indépendamment l'un de l'autre, H, un halogène, un groupe méthyle ou éthyle et X représente Cl ou Br,
caractérisé en ce qu'on fait réagir des chlorures de vinylidène substitués de formule II

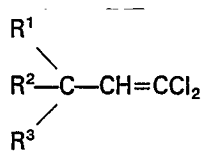

II

dans laquelle
$R^1$ à $R^3$ ont la définition indiquée ci-dessus, à la pression normale ou sous une légère surpression et à des températures comprises entre −20 et +30°C, avec la quantité équivalente de chlore ou de bromure de chlore en présence de composés de formule III

$$R^5{-}SO_3H \qquad III$$

dans laquelle
$R^5$ est un groupe alkyle en $C_1$ à $C_{18}$ éventuellement halogéné ou un groupe aryle éventuellement alkylé.

2. Chlorure d'acide α-halogénopropionique substitué de formule Ia

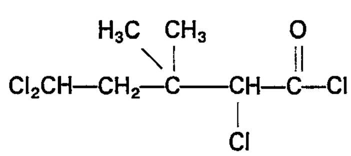

Ia

3. Chlorure de vinylidene substitué de formule IV

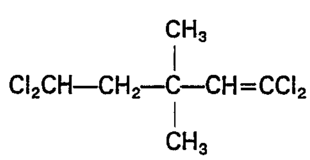

IV

4. Procédé de production de chlorure de vinylidene substitué de formule IV

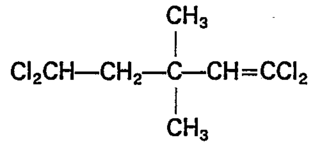

IV

caractérisé en ce qu'on fait réagir le 1,3,3,3-tétrachloro-1,1-diméthylpropane avec la quantité équivalente de chlorure de vinyle en présence d'acides de Lewis.

5. Acide α-halogénopropionique substitué de formule Ib

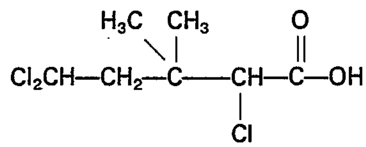

Ib

## Claims

1. Process for the preparation of substituted α-halogenopropionic acid chlorides of the formula

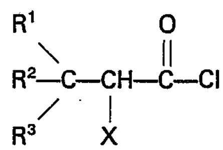

I

in which

$R^1$ represents H, halogen or alkyl; halogen-substituted alkyl or aryl and

$R^2$ and $R^3$ independently of one another represent H, halogen, methyl or ethyl and

X represents Cl or Br,

characterised in that substituted vinylidene chlorides of the formula II

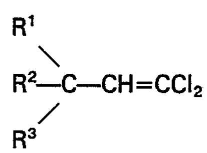

II

in which

$R^1$—$R^3$ have the abovementioned meaning, are reacted, under normal pressure or under a slight excess pressure and at temperatures between −20°C and +30°C, with the equivalent quantity of chlorine or bromine chloride in the presence of compounds of the formula III

$$R^5—SO_3H \qquad III$$

in which

$R^5$ represents optionally halogen-substituted $C_{1-18}$-alkyl or optionally alkyl-substituted aryl.

2. Substituted α-halogenopropionic acid chloride of the formula Ia

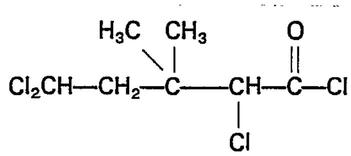

Ia

3. Substituted vinylidene chloride of the formula IV

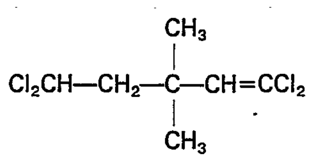

IV

4. Process for the preparation of the substituted vinylidene chloride of the formula IV

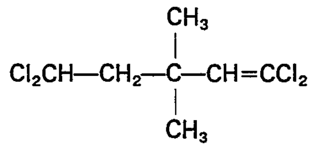

IV

characterised in that 1,3,3,3-tetrachloro-1,1-dimethylpropane is reacted with the equivalent quantity of vinyl chloride in the presence of Lewis acids.

5. Substituted α-halogenopropionic acid of the formula Ib

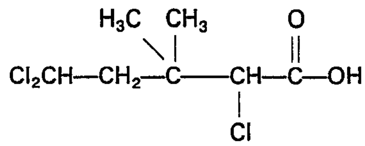

Ib